(19) Europäisches Patentamt
European Patent Office
Office européen des brevets

(11) Publication number: **0 196 769 B1**

(12) # EUROPEAN PATENT SPECIFICATION

(45) Date of publication of patent specification: **08.07.92**

(51) Int. Cl.5: **A61L 15/44**, A61M 37/00

(21) Application number: **86301330.6**

(22) Date of filing: **24.02.86**

The file contains technical information submitted after the application was filed and not included in this specification

(54) **A novel transdermal pharmaceutical absorption dosage unit.**

(30) Priority: **25.02.85 US 705194**
**30.08.85 US 770968**

(43) Date of publication of application:
**08.10.86 Bulletin 86/41**

(45) Publication of the grant of the patent:
**08.07.92 Bulletin 92/28**

(84) Designated Contracting States:
**AT BE CH DE FR GB IT LI LU NL SE**

(56) References cited:
**EP-A- 0 127 468**
**GB-A- 2 081 582**
**GB-A- 2 086 224**
**GB-A- 2 095 108**
**US-A- 3 797 494**
**US-A- 4 316 893**

(73) Proprietor: **Rutgers, The State University of New Jersey**

**New Brunswick New Jersey 08900(US)**

(72) Inventor: **Chien, Yie Wen**
**5, West Lake Court**
**North Brunswick, NJ 08902(US)**
Inventor: **Lee, Chia-Shun**
**17, Forest Glen**
**Highland Park, NJ 08904(US)**

(74) Representative: **Calamita, Roberto et al**
**Frank B. Dehn & Co., Imperial House, 15-19 Kingsway**
**London WC2B 6UZ(GB)**

**EP 0 196 769 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a novel transdermal pharmaceutical absorption dosage unit comprising a backing layer, an intermediate adjoining layer of solid polymer matrix in which the pharmaceutical is dispersed, and a final biologically acceptable adhesive layer which is in communication with the solid polymer matrix layer and is adapted to adhere to the skin of a subject being administered the pharmaceutical, the adhesive layer having dispersed therein an effective amount of one or more skin permeation enhancing compounds for the pharmaceutical dispersed in the polymer matrix layer.

It has been found in the prior art that certain pharmaceuticals are absorbed to a degree through the skin. This is referred to as transdermal pharmaceutical absorption. One means of effecting transdermal absorption has been to distribute the pharmaceutical within a polymeric disc or a container of a gel, which is brought into contact with an area of the skin of the subject to be treated with the pharmaceutical. Also, ointments or lotions containing a desired pharmaceutical have been applied to an area of the skin of the subject to be treated. Problems encountered in such treatment include inadequate control over the rate and duration of the transdermal absorption or the rate can be too slow in the case of certain dosage forms, especially from pharmaceutical-containing discs or pharmaceutical-containing gel container dosage units or pads. It has been found that the transdermal absorption rates of certain pharmaceuticals can be increased by use of absorption-promoting compounds (also referred to as skin permeation enhancers) with the pharmaceutical to be absorbed when compounding the polymeric disc or the pharmaceutical-containing gel.

US-A-3797494 discloses a transdermal dosage unit comprising a backing sheet and a pressure-sensitive adhesive layer, with a reservoir layer containing an active substance, which is sandwiched between the backing sheet and the adhesive layer. Passage of active substance is controlled by a membrane between the adhesive and reservoir layers, possibly with a transport enhancing agent with the active substance in the reservoir layer.

GB-A-2086224 discloses an analgesic composition unit of bi-layer construction having a backing layer together with a single layer comprising a pressure-sensitive adhesive in combination with an analgesic and possibly an absorption accelerating substance.

It is desired to improve the dosage unit forms or devices by which pharmaceuticals are transdermally absorbed, especially in view of the importance of administration of pharmaceuticals by this means. The desired transdermal absorption of pharmaceuticals would provide an avoidance of gastrointestinal incompatibility with the pharmaceuticals and unwanted destruction of the pharmaceutical by metabolism in the gastrointestinal tract and by a "first pass" hepatic metabolism. The transdermal absorption minimizes inter-and intra-patient variations regarding such incompatibilities and metabolisms. By transdermal absorption it is deemed possible to provide more constant pharmaceutical concentration in the body and to realise a greater pharmaceutical efficiency. It is possible, by proper transdermal absorption, to reduce the frequency of effective dosing. Transdermal administration provides most of the advantages of intravenous dosing without the necessity of hospitalization and the accompanying discomfort and inconvenience.

It is desired that improved transdermal pharmaceutical absorption dosage unit forms and processes of transdermal administration be developed. A number of advantages would result.

This invention provides a transdermal pharmaceutical containing matrix dosage unit comprising:

a) a backing layer which is substantially impervious to the pharmaceutical to be delivered transdermally;

b) a polymer matrix disc layer which is adhered to said backing layer and which has micro-dispersed therein an amount of the pharmaceutical which will provide a dosage amount of the pharmaceutical to be delivered transdermally; and

c) an adhesive layer which is adhered to said pharmaceutical-containing polymer matrix disc layer and which has distributed therein an effective amount of one or more skin absorption enhancers which provide substantial skin absorption enhancement for said pharmaceutical;

said dosage unit having an Enhancing Factor, defined as the ratio of normalized permeation rate [in $mcg/cm^2/hr$] of a dosage unit with skin-absorption enhancer to the normalised permeation rate of a dosage unit without enhancer, of at least 1.2.

The backing layer is made from materials that are substantially impermeable with regard to the pharmaceutical of the transdermal dosage unit. It can be made of polymers such as polyethylene, polypropylene, polyvinylchloride, polyesters such as poly(ethylene phthalate), and laminates of polymer films with metallic foils such as aluminium foil.

The polymer matrix disc layer is fabricated from biologically acceptable polymers. The polymer matrix disc layer which has the pharmaceutical distributed therein can suitably be made of a medical-grade silicone polymer such as polydimethylsiloxane polymer. The pharmaceutical is suitably dispersed in the silicone polymer, to which mixture a curing agent is suitably added. The polymer-pharmaceutical mixture is

then formed into a layer of an appropriate thickness and is cured. The matrix layer is adhered to the backing layer which can be done directly. Other suitable polymers which can be used in the formulation of the polymer matrix disc layer are elastomers or thermo-plastics. Care must be taken that the polymer selected is compatible with the pharmaceutical, permits its release for transdermal absorption and is free or sufficiently free from any biologically unacceptable components. The polymer matrix disc layer preferably provides microdispersion compartments having a cross sectional dimension of from about 10 to about 200 microns.

Finally, the adhesive layer is applied to the polymer matrix disc layer. The skin absorption enhancer compound (elsewhere referred to as a skin permeation enhancer) is mixed thoroughly with the adhesive polymer which is suitable for adhesion to the skin locus to which the transdermal matrix delivery dosage unit will be applied. The adhesive polymer-skin permeation enhancer layer can be applied to the polymer matrix disc layer by spraying or by solvent casting. Such a layer is desirably thin, having a thickness in the micron range, preferably having a thickness in the range of from 10 to 200 microns, more preferably from 20 to 180 microns and still more preferably from 30-150 microns.

The transdermal pharmaceutical absorption dosage units of this invention have an Enhancing Factor of at least 1.2, preferably at least 1.3, and more preferably at least about 2.0. Enhancing Factor is defined as the ratio of normalized permeation rate [in $mcg/cm^2/hr$] of a dosage unit of this invention with skin permeation enhancer to the normalized permeation rate of a corresponding dosage unit without enhancer.

The invention thus provides the means for a method of administering a pharmaceutical transdermally by forming a pharmaceutical-containing polymer matrix disc dosage unit having a polymer matrix disc layer which has the pharmaceutical dosage dispersed therein, to which matrix disc is adhered a skin permeation enhancer-containing adhesive layer and, by applying said dosage unit by way of said adhesive layer to the skin of the subject to be treated, whereby said pharmaceutical is transdermally administered systematically to said subject to achieve systemic effects.

The backing layer can be made of any suitable material which is impermeable to the pharmaceutical of the polymer matrix layer. The backing layer serves as a protective cover for the matrix layer and provides also a support function. Examples of materials that are suitable are films of high and low density polyethylene, polypropylene, polyvinylchloride, polyesters such as poly(ethylene phthalate) and the like. Preferably, the materials for the backing layer are laminates of such polymer films with a metal foil such as aluminum foil. In such laminates, a polymer film of the laminate will usually be in contact with the polymer matrix layer. The backing layer can overlay the matrix and adhesive layer as desired for protection and to provide the desired pharmaceutical elegance of the final matrix dosage unit form. The protective layer can be any appropriate thickness which will provide the desired protective and support functions. A suitable thickness will be from about 10 to about 200 microns. Desirably, the thickness will be from about 20 to about 150 microns, and preferably be from about 30 to about 100 microns. The polymer matrix layer can be made from silicone elastomers of the general polydimethylsiloxane structure, such as silicone polymers of the following formula:

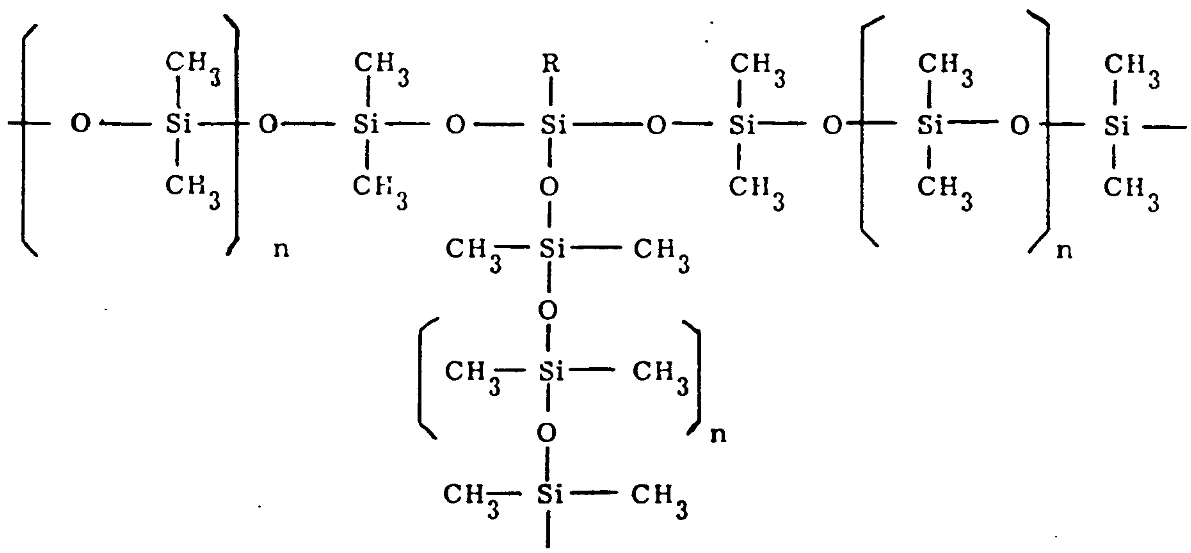

wherein R is alkyl or alkoxy containing 1-7 carbon atoms, vinyl or phenyl and wherein n is about 100-5000.

The silicone polymers selected preferably are cross-linkable at moderate temperatures such as at room temperature, using cross-linking catalysts which are biologically acceptable in the final polymer matrix and which are compatible with the pharmaceutical to be used in making the polymer matrix dosage forms. Some suitable siloxane polymers are cross-linkable copolymers having dimethyl and methylvinyl siloxane units, which can be cross-linked as by using a suitable peroxide catalyst. Other cross-linking sites can be present in the polysiloxane elastomers used. Suitable siloxane medical-grade polymers are sold under the designations Silastic 382, Q7-4635, Q7-4650, Q7-4665, Q7-4735, Q7-4750, Q7-4765 and MDX-4-4210.

Generally, polymers used to form the biologically acceptable polymer matrix are those capable of forming thin walls or coatings through which pharmaceuticals can pass at a controlled rate. Suitable polymers are biologically and pharmaceutically compatible, non-allergenic and insoluble in and non-irritating to body fluids or tissues with which the device is contacted. The use of soluble polymers is to be avoided since dissolution or erosion of the matrix would affect the release rate of the pharmaceutical as well as the capability of the dosage unit to remain in place for convenience of removal.

Exemplary materials for fabricating the biologically acceptable polymer matrix include polyethylene, polypropylene, ethylene/propylene copolymers, ethylene/ethylacrylate copolymers, ethylene/vinyl acetate copolymers, silicone elastomers, especially the medical-grade polydimethylsiloxanes, neoprene rubber, chlorinated polyethylene, polyvinyl chloride; vinyl chloride-vinyl acetate copolymer, polymethacrylate polymer (hydrogel), polyvinylidene chloride, poly(ethylene terephthalate), butyl rubber, epichlorohydrin rubbers, ethylene-vinyl alcohol copolymer, ethylene-vinyloxyethanol copolymer; silicone copolymers, for example, silicone-polycarbonate copolymers; cellulose polymers, for example methyl or ethyl cellulose hydroxypropyl methyl cellulose, and cellulose esters; polycarbonates; polytetrafluoroethylene; and the like. For best results, the biologically acceptable polymer matrix should be selected from polymers with glass transition temperatures below room temperature. The polymer may, but need not necessarily, have a degree of crystallinity at room temperature. Cross-linking monomeric units or sites can be incorporated into such polymers. For example, cross-linking monomers can be incorporated into polyacrylate polymers, which provide sites for cross-linking the matrix after microdispersing the pharmaceutical into the polymer. Known cross-linking monomers for polyacrylate polymers include polymethacrylic esters of polyols such as butylene diacrylate and dimethacrylate, trimethylol propane trimethacrylate and the like other monomers which provide such sites include allyl acrylate, allyl methacrylate, diallyl maleate and the like.

The adhesive layer is suitably made using a silicone adhesive, such as a polydimethylsiloxane adhesive of the following formula:

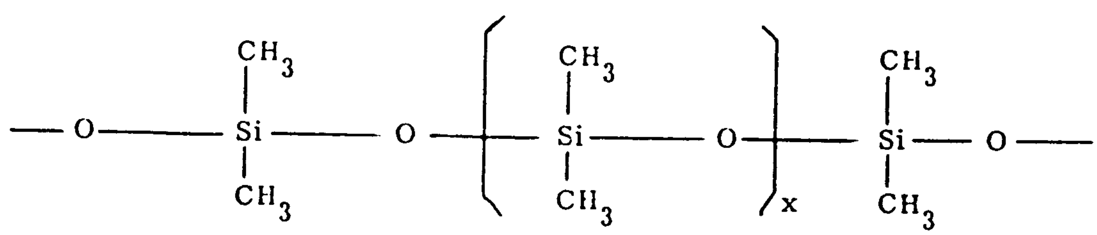

wherein x shows the unit is repeated to the extent to provide desired properties.

For example, adhesive products sold by Dow Corning under the designation DC-355 are suitable for use in making the adhesive layer. The adhesive polymer must be biologically acceptable and compatible with the pharmaceutical and skin permeation enhancer used. Certain polyacrylate adhesive polymers (in the form of an alkyl ester, amide, free acid, or the like) can also be used with some pharmaceuticals. Other suitable hypoallergenic pressure-sensitive contact adhesive compositions are also known. A preferred adhesive layer is pressure-sensitive.

The adhesive layer then is finally covered with a releasable protective layer liner which is made from materials which are substantially impermeable to the pharmaceutical, the skin permeation enhancer used and any other components of the polymer matrix dosage unit. The polymer materials and metal foil laminates used for the backing layer can be used to make the protective layer, provided the layer is made strippable or releasable such as by applying conventional siliconizing.

In making the pharmaceutical-containing polymer matrix disc layer, silicone elastomers such as polydimethylsiloxane of the formula described above can suitably be used. In making nitroglycerin-dispersed polymer matrix disc dosage units, it has been found suitable to use lactose as a dispersing agent. Other suitable dispersing agents can be used instead of lactose. In the case of normally liquid pharmaceutical, a solid-type dispersing agent is suitably used. For pharmaceuticals which are solids, for example those

which are solid between about 25° C and 60° C, water-soluble, high molecular weight polyols, are generally suitable. For example, polyethylene glycols such as those having greater than a molecular weight of 400 can be used. Other suitable dispersing agents known to the formulating art can be used. In the case of some solid pharmaceuticals, it is quite acceptable not to use a dispersing agent. Depending upon the drug loading desired, a suitable amount of a dispersing agent has been found to be 1-9 equivalents (by weight) based on the weight of nitroglycerin. The pharmaceutical then is added to the polymer used to make the matrix disc layer. The amount of the pharmaceutical added depends upon the amount of pharmaceutical dosage desired in each dosage unit and the amount which can be incorporated into the polymer matrix disc to retain suitable structural, diffusion and other properties in the final matrix disc. It has been found, for example, that pharmaceutical can be satisfactorily added to 70 parts of the polymer used in making the matrix disc, such as silicone elastomers. The mixture of the polymer and pharmaceutical is then thoroughly mixed using a high-torque mixer to form a homogeneous microdispersion of the pharmaceutical in the polymer. With continued agitation, an amount of cross-linking catalyst is desirably added together with relatively low molecular weight polymer having a compatible chemical structure. For example, when polydimethylsiloxane is used as the polymer, a relatively low molecular weight polydimethylsiloxane and a cross-linking catalyst is added (such as 10 parts by weight of the low molecular weight polydimethylsiloxane and 30 drops of stannous octanoate per 100g. amount of the final polydimethylsiloxane-pharmaceutical mixture) to the above illustrative composition of 20 parts of pharmaceutical dispersion and 70 parts of polydimethylsiloxane polymer. Again, the mixture is agitated with a high-torque mixer to form a uniform admixture. After each mixing step, the composition is subjected to vacuum to remove any entrapped air.

The deaereated mixture is then placed in a device maker and heated to suitable elevated temperature to promote cross-linking. A suitable temperature for cross-linking when the polymer used is polydimethylsiloxane of the above formula and the cross-linking catalyst is stannous octanoate, is from about 10° C to about 200° C, desirably about 20° C to about 100° C. The temperature used should not cause significant degradation of the pharmaceutical. The polymer matrix sheet desirably is about 0.05 to 5 mm, preferably about 0.1 to about 3 mm in thickness. The resulting cross-linked polymer matrix sheet is removed from the device maker and can be cut to form discs with desired shapes and sizes. The discs are then attached to a backing sheet, as described above, using an adhesive. The discs generally should not exceed about 100 sq. cm. in area, suitably about 5 to 100 sq. cm., preferably, about 8 to about 80 sq. cm., generally about 10 to 60 sq. cm. being more preferable. The shape of the discs can vary; they can be circular, square, rectangular or other desired shape.

The pharmaceutical-containing polymer matrix disc layer, generally speaking, should contain some amount of excess of the dispersed pharmaceutical over the dosage amount desired to be transdermally absorbed by the subject to be treated. Ordinarily, this excess is small, such as less than 2-fold excess. Generally speaking, an amount of the pharmaceutical used, which is sufficient, is less than 2 to about 10 times the desired dosage to about less than 2 to about 5 times the desired dosage to be transdermally absorbed being adequate, depending upon the physiochemical properties of the pharmaceutical, as well as the nature of the polymer of the matrix disc layer and other factors.

The adhesive polymer layer containing the skin permeation enhancer is made as by dissolving the enhancer compound in a solvent for the enhancer which is compatible with the adhesive polymer solution used to make the adhesive layer containing the skin permeation enhancer. Any suitable amount of solvent can be used as necessary to dissolve the quantity of enhancer to be admixed with the adhesive polymer solution used. For example, 3 to 10 parts of solvent can be used to dissolve one part of skin permeation enhancer, depending upon the solubility of the enhancer. When using polydimethylsiloxane adhesive solution, it has been found suitable to use 2 to 20 parts of skin permeation enhancer in 20 to 50 parts of solvent (such as acetone, methyl ethyl ketone, ethyl acetate or other suitable solvent) and add the solution to 100 parts of the adhesive solution. The enhancer - adhesive combination is thoroughly mixed and a coating thereof is applied using a film coating machine to the matrix disk layer or to a strippable release liner, as described above. Preferably, in order to assure adequate adhesion of the adhesive polymer layer to the skin of the subject treated, an enhancer-adhesive polymer solution having a relatively low concentration of enhancer, e.g., 1-2 percent based on the weight of the adhesive polymer is used to apply a coating to the release liner. The thickness of this coating ordinarily is a minor percentage of the thickness of the final adhesive layer, such as 20-40 percent of the total adhesive polymer layer. The remainder of the adhesive polymer layer having a suitable higher concentration of the enhancer is used to coat the matrix disc layer. Suitable higher concentrations of enhancer are usually 10 to about 30 percent based on the adhesive polymer weight, depending upon solubility, desired final amount of skin enhancer agent and other factors. The solvent of the respective coatings is removed by evaporation. The respective coatings are combined to make the final adhesive polymer-enhancer agent layer by application of constant pressure.

A suitable release liner being a poly(ethylene phthalate) laminated with aluminum foil. The poly(ethylene phthalate) side to which the adhesive - enhancer coating is applied, is made strippable by conventional siliconizing or by other suitable means. The thickness of the adhesive - enhancer layer normally is suitably about 10 to about 200 microns, preferably about 30 to about 150 microns. The amount of enhancer in the adhesive layer depends in part on the rapidity at which it is desired that the pharmaceutical be absorbed. Generally speaking, about 1 to about 30 percent of skin permeation enhancer based on the weight of the adhesive is suitable depending upon the enhancer, matrix polymer, adhesive and other factors. Desirably, about 2 to about 20 percent of skin permeation enhancers are used depending upon the above recited factors. The adhesive layer containing the skin permeation enhancer is applied to the polymer matrix disc surfaces by application of a constant pressure.

The four-layer transdermal pharmaceutical polymer matrix dosage units are excised. The backing layer as desired can be shaped around the sides of the dosage unit including the polymer matrix layer if such protection is desired. The resulting pharmaceutical polymer matrix dosage unit forms are then placed in appropriate storage until they are to be applied in transdermal treatment.

At least one pharmaceutical is dispersed in the polymer matrix disc layer. The type of pharmaceutical which may be dispersed in the polymer matrix disc layer includes any pharmaceutical which is capable of being transdermally or topically administered to a subject to be treated. The pharmaceutical used should have a daily effective dose of less than about 100 mg. With the controlled release of the pharmaceutical at a relatively steady rate over a prolonged period, typically 24 hours or longer, the patient is provided with the benefit of a steady infusion of the pharmaceutical over a prolonged period. As examples of pharmaceuticals which can be included in the polymer matrix disc layer of the present invention, there may be mentioned the following: alpha-[1(methylamino)ethyl]-benzene methanol, which is useful as an adrenergic (bronchodilator); N-phenyl-N-[1-(2-phenylethyl)-4-piperidinyl] propanamide, useful as a narcotic analgesic; furosemide and 6-chloro-3, 4-dihydro-2H-1, 2,4-benzothiadiazine-7-sulfonamide 1,1-dioxide, useful as diuretics; and 2-diphenylmethoxy-N, N-dimethylethanamine, useful as an antihistamine. Other useful drugs include: antimicrobial agents such as penicillins, cephalosporins, tetracycline, oxytetracycline, chlortetracycline, chloramphenicol; and sulfonamides, sedatives and hypnotics such as pentabarbital, sodium pentabarbital, secobarbital sodium, codeine (abromoisovaleryl) urea, and carbromal; psychic energizers such as 3-(2-aminopropyl) indole acetate and 3-(2-aminobutyl) indole acetate; tranquilizers such as diazepam, chlordiazepoxide hydrochloride, reserpine, chlorpromazine hydrochloride, and thiopropazate hydrochloride; hormones such as adrenocorticosteroids, for example, 6-methyl-prednisolone; androgenic steroids, for example, testosterone, methyltestosterone, and fluoxymesterone; estrogenic steroids, for example, estrone, estradiol and ethinyl estradiol; progestational steroids, for example, 17a-hydroxyprogesterone and acetate, medroxyprogesterone and acetate, 19-norprogesterone, and norethindrone; thyroxine; antipyretics such as aspirin, salicylamide, methylsalicylate triethanolamine and salicylate; morphine and other narcotic analgesics; hypoglycemic agents, for example, sulfonyl ureas such as glypizide, glyburide and chlorpropamide and insulin; antispasmodics such as atropine, methscopolamine bromide, methscopolamine bromide with phenobarbital; antimalarials such as the 4-aminoquinolines, 9-aminoquinolines, and pyrimethamine; adrenergic block agent such as metoprolo; antiarthritic agents such as sulindac; nonsteroidal anti-inflammatory agents such as ibuprofen and naproxen; vasodilators such as dipyridamole, isosorbide dinitrate; antihypertension agents such as propranolol, methyldopa and prazosin; contraceptive agents such as levonorgestrel/estradiol combination and ethynoldiol diacetate/estrogen combination; agents for treating duodenal ulcers such as cimetidine; and nutritional agents such as vitamins, essential amino acids, and essential fats. The above listing of pharmaceuticals is merely exemplary of the transdermally applicable pharmaceuticals. It is contemplated that any pharmaceutical can be utilized may be transdermally administered by use of this invention.

It will be appreciated that the pharmaceutical may be added to the above mixture not only in the form of the pure chemical compound, but also in admixture with other pharmaceuticals which may be transdermally applied or with other ingredients which are not incompatible with the desired objective of transdermally administering the pharmaceutical to a patient. Thus, simple pharmacologically acceptable derivatives of the pharmaceuticals such as ethers, esters, amides, acetals, salts and the like may be used. In some cases, such derivatives may actually be preferred.

The skin permeation enhancers which can be used in carrying out this invention can vary. Ones that give preferred results with the polymer matrix dosage unit form having a specific pharmaceutical can vary. In some instances the use of permeation enhancer in making a polymer matrix dosage form will result in good or even excellent absorption for one pharmaceutical but may result in no or relatively low enhancement when another pharmaceutical is used. Use of combinations of two or more skin permeation enhancer compounds frequently results in superior results, such as greater transdermal absorption.

Specific skin permeation enhancers which can be used in making the polymer matrix dosage forms of this invention include saturated and unsaturated fatty acids and their esters (e.g. alkyl esters), alcohols, monoglycerides, glycylglycine, diethanolamides, triethanolamine complexes and N, N-dimethylamides, derivates and acetate esters, examples being oleic acid, propyl oleate, monoolein,oleyl acetate, propyl myristate,mono-myristein, myristyl alcohol, oleyl alcohol, myristyl N, N-dimethyl amide, stearic acid and stearyl alcohol, propyl stearate,monostearin, and combinations of these with, for example, 1-dodecylazacycloheptan-2-one sold under the trademark Azone by Nelson Research and Development; decyl methyl sulfoxide, dimethyl sulfoxide, salicyclic acid derivatives, N,N-diethyl-m-toluamide, crotamiton, 1-substituted azacycloalkan-2-ones such as disclosed in U.S. Patent 4,316,893 for example compounds of the formula:

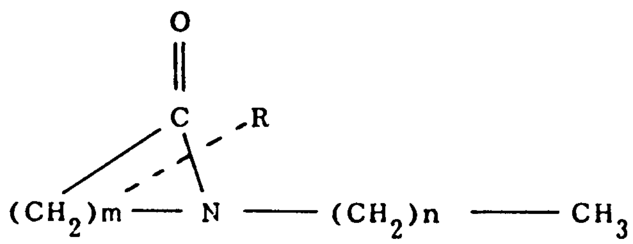

wherein R is H or a lower alkyl group, m is 5-7 and n is 0-17, preferably 1-11, and various other compounds which are biologically compatible and have trnsdermal permeation activity. Ethyl alcohol is ineffective or substantially ineffective for enhancing absorption in compositions according to the invention and therefore ethyl alcohol and other short chain alkanols (with 1-4 carbon atoms) which have substantially the same properties and activity as ethyl alcohol do not come within the term "skin absorbing enhancer" as used herein.

The invention further provides a method of producing a transdermal pharmaceutical-containing polymer matrix dosage unit having an Enhancing Factor as herein defined of at least 1.2, which method comprises:

(a) adhering one side of a polymer matrix disc layer which has microdispersed therein an amount of the pharmaceutical which will provide a dosage amount of the pharmaceutical to be delivered transdermally to a backing layer which is substantially impervious to said pharmaceutical, and

(b) adhering the other side of said polymer matrix disc layer to an adhesive layer which has distributed therein an effective amount of one or more skin absorption enhancers which provide substantial skin absorption enhancement for said pharmaceutical.

The following examples illustrate the invention and are not intended to be limiting.

EXAMPLE 1

A dispersion of 10 parts by weight each of pure nitroglycerin oil and lactose is made using a high-torque mixer (sold by Cole-Parmer Company). The nitroglycerin-lactose dispersion is homogeneously dispersed in 70 parts of silicone elastomer using the high-torque mixer and about 1,000 RPM. The silicone elastomer is a polydimethylsiloxane polymer sold by Dow-Corning Company under the designation Silastic Medical Grade 382 Elastomer. The elastomer is believed to have the following structural formula:

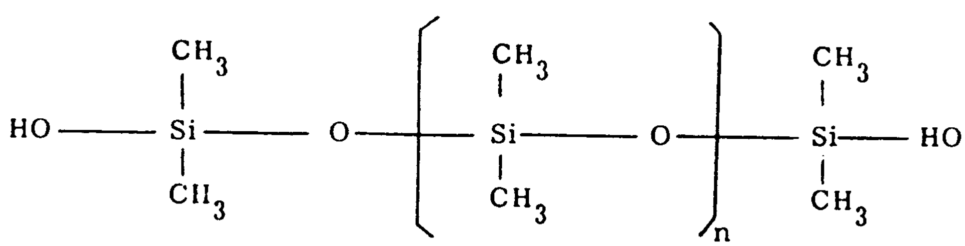

wherein n indicates the number of repeating units.

With continued agitation, 10 parts of DC-360 (silicone medical fluid) and 30 drops (for every 100 g of the mixture) of a cross-linking agent designated as catalyst M is added, which is stannous octanoate. After each addition, of the mixture, material is thoroughly mixed, and the dispersed mixture is placed under vacuum to remove entrapped air.

The nitroglycerin-polydimethylsiloxane dispersion is placed into a device maker and is cross-linked at an elevated temperature (25°-60°C) to form a cross-linked, medicated polymer sheet, which has a thickness of 0.2-2 mm.

The medicated polymer sheet is removed from the device maker and is cut into circular discs of about 3-20 sq.cm. The discs are attached to a backing layer of heat sealable polyester film which is laminated to aluminum foil. This laminate is sold by 3M Company as Scotchpak 1006. The medicated discs are attached using an adhesive polymer solution, which is a silicone adhesive polymer sold by Dow Corning as DC-355. Alternately, the discs can be formed directly on the backing layer and in practice are.

The skin permeation enhancer-adhesive layer is made by dissolving 6.5 parts by weight of a skin permeation enhancer in 30 parts of acetone. The acetone solution then is added to 100 parts of a silicone adhesive solution sold by Dow-Corning under the designation of DC-355.

The mixture is thoroughly mixed to form a homogeneous mixture of skin permeation enhancer and adhesive polymer, which is applied to a strip of a release liner which is a siliconized polyester film to permit easy removal of the film just prior to application of the final polymer matrix disc dosage unit to the subject to be transdermally treated. The adhesive mixture is applied at a controlled thickness. The formed layer has a thickness of about 50-200 microns. The layer is dried completely in vacuum to remove volatile matter.

The skin permeation enhancer-adhesive polymer layer with release liner is applied onto the pharamceutical-containing polymer matrix disc with the attached backing layer under a constant pressure to provide a firmly adhered strip of a four-layered structure as follows:

1. Backing layer
2. Pharmaceutical-containing polymer matrix layer.
3. Skin permeation enhancer-adhesive layer
4. Release film layer which can be readily removed to permit application to the skin of the subject to receive transdermally the nitroglycerin.

By use of an appropriate cutter, the strip is cut to provide the transdermal nitroglycerin polymer matrix dosage units which are circular in shape and have an area of about 20 sq. cm.

The above polymer matrix disc dosage units are made using the following skin permeation enhancers: oleic acid; propyl oleate; decyl methyl sulfoxide; and 1-dodecylazacycloheptan-2-one.

Use of tert-butyl alcohol did not function as a skin permeation enhancer but rather the dosage units using tert-butyl alcohol resulted in less nitroglycerin absorption than the absorption from a controlled unit in which no enhancer compound was added to the adhesive layer.

The transdermal absorption of the pharmaceutical from the pharmaceutical polymer matrix dosage units of this invention is evaluated by use of skin from a "hairless" mouse or human cadaver by following the procedure described by P.R. Keshary and Y.W. Chien, in Drug Develop. & Ind. Pharm., 10 (6) 883-913 (1984).

Table I shows the transdermal absorption of nitroglycerin from the nitroglycerin-containing polymer matrix disc dosage units made by the above procedure. Table II shows the effect of the skin permeation enhancer concentration in the adhesive layer on the enhancement of transdermal absorption of nitroglycerin and Table III shows the skin permeation enhancement of nitroglycerin by enhancers in combination.

TABLE I

TRANSDERMAL ABSORPTION FROM POLYMER MATRIX DISC DOSAGE UNITS OF ANTI-ANGINAL DRUG - NITROGLYCERIN

| Enhancers(1) (1.6 MG/CM$^2$) | Normalized Permeation Rate (MCG/CM$^2$/HR ± S.D.) | Enhancing Factors(2) |
|---|---|---|
| None | 25.72 ± 5.41 | 1.00 |
| Stearic Acid | 27.26 ± 2.53 | 1.06 |
| Stearyl Alcohol | 25.38 ± 0.05 | 0.99 |
| Stearyl Propyl Ester | 42.91 ± 4.81 | 1.67 |
| Mono-Stearin | 36.82 ± 0.01 | 1.43 |
| Oleic Acid | 70.55 ± 11.06 | 2.74 |
| Oleyl Alcohol | 61.74 ± 5.83 | 2.40 |
| Oleyl Propyl Ester | 62.40 ± 11.12 | 2.43 Mono-Olein 51.14 |
| ± 10.52 | 1.99 | |
| Myristic Acid | 28.04 ± 1.33 | 1.09 |
| Myristyl Alcohol | 45.72 ± 2.33 | 1.78 |
| Myristyl Propyl Ester | 49.46 ± 5.62 | 1.92 |
| Mono-Myristein | 22.54 ± 2.90 | 0.88 |
| 1-Dodecylazacycloheptan-2-One | 35.83 ± 3.90 | 1.39 |
| Decyl Methyl Sulfoxide | 56.68 ± 1.82 | 2.20 |
| Tert-Butanol | 22.73 ± 2.02 | 0.88 |

1) Contained in the Adhesive Layer at a Surface Concentration of 1.6 MG/CM$^2$

2) Enhancing Factor = $\frac{\text{(Normalized Permeation Rate) Enhancer}}{25.72}$

TABLE II

EFFECT OF SKIN PERMEATION ENHANCER CONCENTRATION IN ADHESIVE LAYER ON THE ENHANCEMENT OF TRANSDERMAL ABSORPTION OF NITROGLYCERIN

| Enhancing Conc. | Enhancing Factor | | | | |
|---|---|---|---|---|---|
| ($mg/cm^2$) | A* | B | C | D | E |
| 0 | 1.00 | 1.00 | 1.00 | 1.00 | 1.00 |
| 0.08 | 1.16 | -- | -- | -- | -- |
| 0.20 | 1.41 | -- | -- | -- | -- |
| 0.37 | 1.68 | -- | -- | -- | -- |
| 0.66 | 2.41 | -- | -- | -- | -- |
| 0.80 | -- | 1.67 | 1.22 | 1.56 | 1.35 |
| 0.96 | 1.99 | -- | -- | -- | -- |
| 1.27 | 1.41 | -- | -- | -- | -- |
| 1.59 | 1.44 | 1.92 | 2.43 | 2.58 | 2.02 |
| 3.18 | 1.51 | 3.07 | 3.08 | 1.50 | 1.66 |
| 4.78 | -- | 3.41 | 3.78 | 1.74 | 1.54 |

*Enhancer A = 1-Dodecylazacycloheptan-2-one
B = propyl myristate
C = propyl oleate
D = 1-monolauroyl-rac-glycerol
E = Myristic acid, N,N-dimethylamide

TABLE III
SKIN PERMEATION ENHANCEMENT OF NITROGLYCERIN BY ENHANCERS IN COMBINATION

| Skin Permeation Enhancers (mg/cm²) | | | Enhancing Factor |
|---|---|---|---|
| A* | B | C | |
| 1.27 | -- | -- | 1.41 |
| 1.27 | 1.27 | -- | 1.63 |
| 1.27 | -- | 1.27 | 2.15 |
| -- | 1.27 | 1.27 | 1.28 |
| 1.27 | 1.27 | 1.27 | 2.37 |

*Enhancer A = 1-Dodecylazacyloheptan-2-one
B = propyl myristate
C = propyl oleate

EXAMPLE 2

The following dosage units are made and evaluated, using the general procedure set out in Example 1. No dispersing agent is employed in fabricating the polymer matrix discs.

TABLE IV

TRANSDERMAL ABSORPTION FROM POLYMER MATRIX DISC DOSAGE UNITS OF ANTI-ARRHYTHMIC DRUG - VERAPAMIL

| Enhancers (3.2 MG/CM²) | Normalized Permeation Rate (MCG/CM²/HR ± S.D.) | Enhancing Factors |
|---|---|---|
| None | 4.58 ± 1.55 | 1.00 |
| Propyl Myristate | 9.03 ± 2.40 | 1.97 |
| Propyl Oleate | 11.70 ± 2.25 | 2.73 |
| 1-Dodecylazacycloheptan-2-One | 11.55 ± 2.04 | 2.52 |

EXAMPLE 3

The following dosage units are made following the general procedure of Example 1. The following ingredients are used in making the pharmaceutical-containing polymer matrix discs: pharmaceutical, 10

parts; DC-360 polysiloxane medical fluid (20 cps), 10 parts; silicone (medical-grade) elastomer, 80 parts, and catalyst M (20 drops/100g. of mixture).

The pharmaceutical crystals (10 parts) are dispersed in 10 parts of DC-360 silicone medical fluid to a uniform dispersion. The pharmaceutical dispersion is homogeneously dispersed in 80 parts of Silastic medical-grade 382 elastomer.

The skin permeation enhancer-adhesive film is made using the following ingredients: skin permeation enhancer, 6.5 parts; solvent, 30 parts; and adhesive polymer solution; 100 parts.

Transdermal polymer matrix dosage units are obtained and evaluated as shown in Tables V-XI.

TABLE V

TRANSDERMAL ABSORPTION FROM POLYMER MATRIX DISC DOSAGE UNITS OF PROGESTATIONAL STEROID - PROGESTERONE

| Enhancers (3.2 $MG/CM^2$) | Normalized Permeation Rate ($MCG/CM^2/HR \pm S.D.$) | Enhancing Factors |
|---|---|---|
| None | 1.53 ± 0.43 | 1.00 |
| Propyl Myristate | 6.98 ± 1.33 | 4.56 |
| Propyl Oleate | 8.20 ± 1.72 | 5.36 |
| 1-Dodecylazacycloheptan-2-One | 9.12 ± 3.53 | 5.96 |

TABLE VI

TRANSDERMAL ABSORPTION FROM POLYMER MATRIX DISC DOSAGE UNITS OF ANTI-INFLAMMATORY STEROID - HYDROCORTISONE

| Enhancers (3.2 MG/$CM^2$) | Normalized Permeation Rate (MCG/$CM^2$/HR ± S.D.) | Enhancing Factors |
|---|---|---|
| None | 0.046 ± 0.005 | 1.00 |
| Propyl Myristate | 0.21 ± 0.073 | 4.57 |
| Propyl Oleate | 0.23 ± 0.045 | 5.00 |
| 1-Dodecylazacycloheptan-2-One | 2.82 ± 0.58 | 61.30 |

TABLE VII

TRANSDERMAL ABSORPTION FROM POLYMER MATRIX DISC DOSAGE UNITS OF ANALGESIC DRUG - HYDROMORPHONE

| Enhancers (3.2 MG/$CM^2$) | Normalized Permeation Rate (MCG/$CM^2$/HR ± S.D.) | Enhancing Factors |
|---|---|---|
| None | 0.103 ± 0.022 | 1.00 |
| Propyl Myristate | 0.41 ± 0.11 | 3.98 |
| Propyl Oleate | 0.37 ± 0.098 | 3.59 |
| 1-Dodecylazacycloheptan-2-One | 0.94 ± 0.35 | 9.13 |

TABLE VIII

TRANSDERMAL ABSORPTION FROM POLYMER MATRIX DISC DOSAGE UNITS OF ANTI-ARTHRITIC DRUG - INDOMETHACIN

| Enhancers ($3.2 MG/CM^2$) | Normalized Permeation Rate ($MCG/CM^2/HR \pm S.D.$) | Enhancing Factors |
|---|---|---|
| None | 0.39 ± 0.08 | 1.00 |
| Propyl Myristate | 1.47 ± 0.10 | 3.77 |
| Propyl Oleate | 1.82 ± 0.39 | 4.67 |
| 1-Dodecylazacycloheptan-2-One | 5.65 ± 0.89 | 14.49 |

TABLE IX

TRANSDERMAL ABSORPTION FROM POLYMER MATRIX DISC DOSAGE UNITS OF ANTI-ARTHRITIC DRUG - INDOMETHACIN BY USING COMBINATION OF ENHANCERS

| Enhancers* ($3.2\ MG/CM^2$) | Normalized Permeation Rate ($MCG/CM^2/HR \pm S.D.$) | Enhancing Factors$^\Delta$ |
|---|---|---|
| None | 0.32 ± 0.08 | 1.00 |
| Oleic Acid | 4.31 ± 0.85 | 13.47 |
| Oleyl Alcohol | 4.88 ± 0.61 | 15.25 |
| Oleyl Acetate | 6.07 ± 0.65 | 18.96 |
| Propyl Oleate | 2.70 ± 0.002 | 8.44 |
| Monoolein | 2.30 ± 0.42 | 7.19 |
| Propyl Myristate | 2.87 ± 0.55 | 8.98 |
| Myristyl Alcohol | 1.63 ± 0.32 | 5.10 |
| Myristyl N,N-Dimethyl Amide | 3.85 ± 0.07 | 12.02 |

*The adhesive layer contains equal concentrations of 1-dodecylazacyclo-heptan-2-one and second named enhancer. $^\Delta$The enhancing factor for 1-dodecylazacyclo-heptan-2-one alone ($1.6\ MG/CM^2$) is 7.48.

TABLE X

TRANSDERMAL ABSORPTION FROM POLYMER MATRIX DISC DOSAGE UNITS OF ANDROGENIC STEROID - TESTOSTERONE

| Enhancers | Conc. ($mg/cm^2$) | Normalized Permeation Rate ($mcg/cm^2/hr$ ±SD) | Enhancing Factor |
|---|---|---|---|
| None | 0.00 | 0.57 ± 0.04 | 1.00 |
| N,N-Diethyl-m toluamide | 1.60<br>3.20 | 1.06 ± 0.06<br>2.61 ± 0.11 | 1.86<br>4.58 |
| 1-Dodecylazacycloheptan-2-one | 1.60 | 4.29 ± 2.28 | 7.53 |
| 1-Dodecylazacycloheptan-2-one+<br>Oleyl alcohol | 1.60<br>1.60 | 4.37 ± 0.72 | 7.67 |
| 1-Dodecylazacycloheptan-2-one+<br>Myristyl-N,N-dimethylamide | 1.60<br>1.60 | 7.62 ± 0.92 | 13.37 |
| 1-Dodecylazacycloheptan-2-one+<br>Decylmethylsulfoxide | 1.60<br>1.60 | 8.92 ± 0.96 | 15.65 |

TABLE XI

TRANSDERMAL ABSORPTION FROM POLYMER MATRIX DISC DOSAGE UNITS CONTAINING DECYL METHYL SULFOXIDE AS SKIN ABSORPTION ENHANCER

| Drugs | Enhancer Conc. ($MG/CM^2$) | Permeation Rate ($MCG/CM^2/HR$ ± S.D.) | Enhancing Factors |
|---|---|---|---|
| Nitroglycerin | 0 | 34.48 ± 7.03 | 1.00 |
| | 5 | 46.74 ± 1.74 | 1.36 |
| | 10 | 41.91 ± 0.06 | 1.22 |
| Indomethacin | 0 | 0.49 ± 0.22 | 1.00 |
| | 5 | 2.68 ± 0.30 | 5.47 |
| | 10 | 7.68 ± 0.31 | 15.67 |
| Progesterone | 0 | 1.11 ± 0.68 | 1.00 |
| | 5 | 8.02 ± 0.14 | 7.23 |
| | 10 | 12.25 ± 0.61 | 11.04 |
| Hydrocortisone | 0 | 0.065 ± 0.001 | 1.00 |
| | 5 | 0.72 ± 0.094 | 11.08 |
| | 10 | 1.64 | 25.23 |
| Verapamil | 0 | 3.45 ± 0.55 | 1.00 |
| | 5 | 8.14 ± 1.06 | 2.36 |
| | 10 | 11.73 ± 3.07 | 3.40 |
| Hydromorphone | 0 | 0.103 ± 0.022 | 1.00 |
| | 5 | 0.43 ± 0.008 | 4.17 |
| | 10 | 0.95 ± 0.22 | 9.22 |

EXAMPLE 4

The following dosage units are made following the general procedure of Example 1. The following ingredients are used in making the pharmaceutical-containing polymer matrix discs: estradiol, 5 parts; aqueous solution of polyethylene gylcol-mol. wt.400 (40% v/v), 20 parts DC-360 polysiloxane medical fluid (20 cps), 5 parts; silicone (medical-grade) elastomer, 70 parts; catalyst M, 20 drops per 100g. of the mixture.

The estradiol crystals are thoroughly dispersed in the polyethylene glycol solution, which is homogeneously dispersed in the 70 parts of Silastic medical-grade 382 elastomer.

The skin permeation enhancer-adhesive film is made using the following ingredients: skin permeation enhancer, 6.5 parts; solvent 30 parts; and adhesive polymer solution, 100 parts.

Transdermal polymer matrix dosage units are obtained and evaluated as shown in Table XII.

TABLE XII

TRANSDERMAL ABSORPTION FROM POLYMER MATRIX DISC DOSAGE UNITS OF ESTROGENIC STEROID - ESTRADIOL

| Enhancers (3.2 $MG/CM^2$) | Normalized Permeation Rate ($MCG/CM^2/HR \pm S.D.$) | Enhancing Factors |
|---|---|---|
| None | 1.22 ± 1.06 | 1.00 |
| Propyl Myristate | 11.38 ± 3.38 | 9.33 |
| Propyl Oleate | 17.84 ± 8.20 | 14.62 |
| 1-Dodecylazacycloheptan-2-One | 24.61 ± 8.93 | 20.17 |
| Decyl Methyl Sulfoxide | 15.36 ± 0.45 | 12.59 |

## Claims

**1.** A transdermal pharmaceutical-containing polymer matrix dosage unit comprising:

a) a backing layer which is substantially impervious to the pharmaceutical to be delivered transdermally;

b) a polymer matrix disc layer which is adhered to said backing layer and which has microdispersed therein an amount of the pharmaceutical which will provide a dosage amount of the pharmaceutical to be delivered transdermally; and

c) an adhesive layer which is adhered to said pharmaceutical-containing polymer matrix disc layer and which has distributed therein an effective amount of one or more skin absorption enhancers which provide substantial skin absorption enhancement for said pharmaceutical;

said dosage unit having an Enhancing Factor, defined as the ratio of normalized permeation rate [in $mcg/cm^2/hr$] of a dosage unit with skin-absorption enhancer to the normalised permeation rate of a dosage unit without enhancer, of at least 1.2.

**2.** A transdermal pharmaceutical-containing polymer matrix dosage unit as claimed in claim 1 wherein the polymer matrix disc layer is a cross-linked polysiloxane polymer of the following formula:

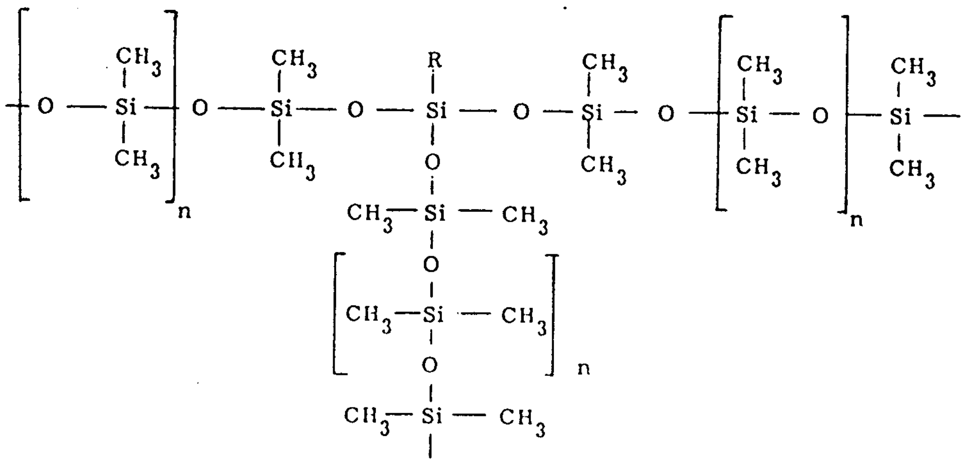

wherein R is alkyl or alkoxy having 1-7 carbon atoms, vinyl, phenyl or a combination thereof and wherein n is about 100 to about 5,000.

**3.** A transdermal pharmaceutical-containing polymer matrix dosage unit as claimed in claim 2 wherein R in the polysiloxane formula is propoxy.

**4.** A transdermal pharmaceutical-containing polymer matrix dosage unit as claimed in any of claims 1 to 3 wherein the polymer matrix disc layer has a thickness of from 0.05 to 5mm.

**5.** A transdermal pharmaceutical-containing polymer matrix dosage unit as claimed in any of claims 1 to 4 wherein the polymer matrix disc provides microdispersion compartments having a cross sectional dimension of from aobut 10 to about 200 microns.

**6.** A transdermal pharmaceutical-containing polymer matrix dosage unit as claimed in any of claims 1 to 5 wherein the adhesive layer is made from a pressure-sensitive adhesive.

**7.** A transdermal pharmaceutical-containing polymer matrix dosage unit as claimed in any of claims 1 to 6 wherein the adhesive layer is made from a silicone polymer adhesive.

**8.** A transdermal pharmaceutical-containing polymer matrix dosage unit as claimed in any of claims 1 to 7 wherein the skin absorption enhancer is selected from decyl methyl sulphoxide; 1-dodecylazacycloheptan-2-one; a compound of formula:

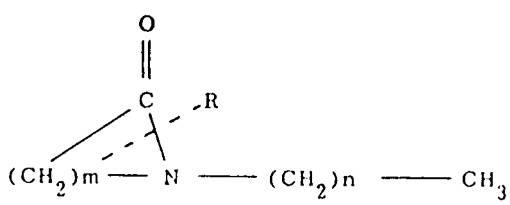

wherein R is H or a lower aklyl group, n is 5-7 and n is 0-17, saturated and unsaturated fatty acids and their alkyl esters, alcohols, monoglycerides, diethanolamides, triethanolamine complexes, and N, N-dimethylamide derivatives, and acetate esters of said alcohol derivatives; propyl myristate; myristyl alcohol; propyl oleate; oleic acid; oleyl alcohol; oleyl acetate; monoolein; myristyl N, N-dimethyl amide; stearic acid; stearyl alcohol; propyl stearate; mono-stearin; mono-myristein; salicylic acid and derivatives; glycylglycine; N N-diethyl-m-toluamide; crotamiton; and combinations thereof.

**9.** A transdermal pharmaceutical-containing polymer matrix dosage unit as claimed in any of claims 1 to 8 wherein the adhesive layer has distributed therein from about 1 to about 30 per cent of one or more skin absorption enhancers based on the weight of the polysiloxane adhesive.

**10.** A transdermal pharmaceutical-containing polymer matrix dosage unit as claimed in any of claims 1 to 9 wherein the Enhancing Factor as herein defined is at least about 2.0.

**11.** A transdermal pharmaceutical-containing polymer matrix dosage unit as claimed in any of claims 1 to 10 which has a releasable protective layer adhered to the adhesive layer.

**12.** A transdermal pharmaceutical-containing polymer matrix dosage unit as claimed in any of claims 1 to 11 wherein the pharmaceutical has anti-anginal, anti-arrhythmic, anti-hypertensive, tranquillizing, diuretic, central nervous system, anti-inflammatory, analgesic or anti-arthritic activity.

**13.** A transdermal pharmaceutical-containing polymer matrix dosage unit as claimed in any of claims 1 to 12 wherein the pharmaceutical is a steroid.

**14.** A transdermal pharmaceutical-containing polymer matrix dosage unit as claimed in claim 13, wherein the pharmaceutical comprises testosterone, estradiol, progesterone, hydrocortisone or Indomethacin.

**15.** A transdermal pharmaceutical-containing polymer matrix dosage unit as claimed in any of claims 1 to 12 wherein the pharmaceutical is nitroglycerin.

**16.** A method a producing a transdermal pharmaceutical-containing polymer matrix dosage unit having an Enhancing Factor as defined in claim 1 of at least 1.2, which method comprises:
(a) adhering one side of a polymer matrix disc-layer which has microdispersed therein an amount of the pharmaceutical which will provide a dosage amount of the pharmaceutical to be delivered transdermally to a backing layer which is substantially impervious to said pharmaceutical, and
(b) adhering the other side of said polymer matrix disc layer to an adhesive layer which has distributed therein an effective amount of one or more skin absorption enhancers which provide substantial skin absorption enhancement for said pharmaceutical.

**Revendications**

**1.** Unité de dosage à matrice polymère contenant un produit pharmaceutique transdermique comprenant :
a) une couche de renforcement qui est substantiellement imperméable au produit pharmaceutique devant être administré par voie transdermique;
b) une couche de matrice polymère en forme de disque qui adhère à ladite couche de renforcement et comporte une quantité du produit pharmaceutique en microdispersion qui fournira une quantité de dosage du produit pharmaceutique devant être administré par voie transdermique; et
c) une couche adhésive qui adhère à ladite couche de matrice polymère en forme de disque contenant le produit pharmaceutique et comporte une quantité efficace distribuée en son sein d'un ou plusieurs agents d'amélioration d'absorption de la peau qui procure une amélioration substantielle de l'absorption de la peau pour ledit produit pharmaceutique;
ladite unité de dosage présentant un Facteur d'Amélioration, défini comme le rapport du taux de pénétration normalisée (en $mcg/cm^2/h$) d'une unité de dosage comportant un agent d'amélioration de l'absorption de la peau au taux de pénétration normalisée d'une unité de dosage ne comportant pas l'agent d'amélioration, d'au moins 1,2.

**2.** Unité de dosage à matrice polymère contenant un produit pharmaceutique transdermique selon la revendication 1 dans laquelle la couche de matrice polymère sous forme de disque consiste en un polysiloxane réticulé de formule suivante :

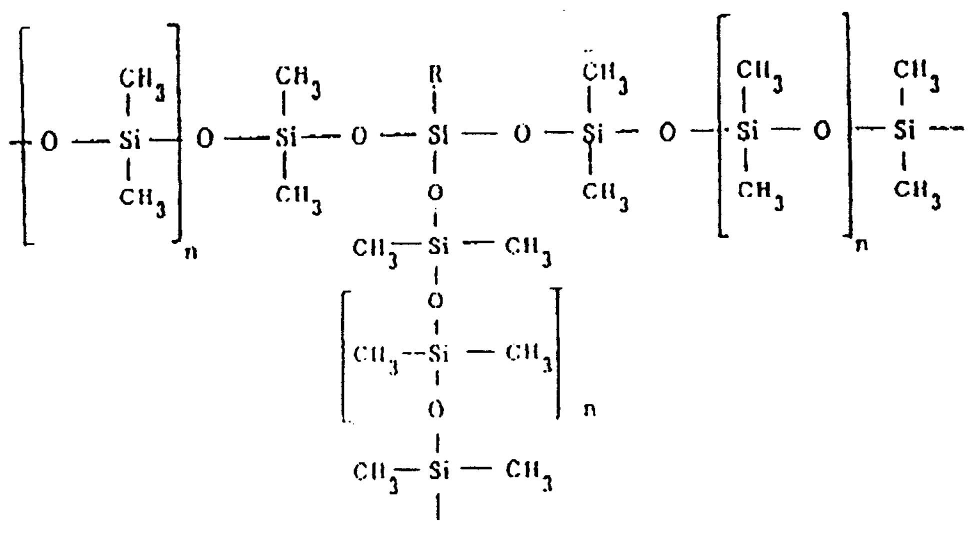

dans laquelle R est un groupe alkyle ou alcoxy de 1 à 7 atomes de carbone, un groupe vinyle, un groupe phényle ou une combinaison de ceux-ci et dans laquelle n est compris entre environ 100 et environ 5000.

3. Unité de dosage à matrice polymère contenant un produit pharmaceutique transdermique selon la revendication 2 dans laquelle R, dans la formule du polysiloxane, est un groupe propoxy.

4. Unité de dosage à matrice polymère contenant un produit pharmaceutique transdermique selon l'une quelconque des revendications 1 à 3 dans laquelle la couche de matrice polymère en forme de disque a une épaisseur comprise entre 0,05 et 5mm.

5. Unité de dosage à matrice polymère contenant un produit pharmaceutique transdermique selon l'une quelconque des revendications 1 à 4 dans laquelle le disque de matrice polymère procure des compartiments de micro-dispersion ayant une dimension en coupe transversale comprise entre environ 10 et environ 200 microns.

6. Unité de dosage à matrice polymère contenant un produit pharmaceutique transdermique selon l'une quelconque des revendication 1 à 5 dans laquel le la couche adhésive est constituée d'un adhésif sensible à la pression.

7. Unité de dosage à matrice polymère contenant un produit pharmaceutique transdermique selon l'une quelconque des revendications 1 à 6 dans laquelle la couche adhésive est constituée d'un adhésif polymère de silicone.

8. Unité de dosage à matrice polymère contenant un produit pharmaceutique transdermique selon l'une quelconque des revendications 1 à 7 dans laquelle l'a gent d'amélioration d'absorption de la peau est choisi parmi le décyl méthyl sulfoxyde; le 1-dodécylazacycloheptane-2-one; un composé de formule :

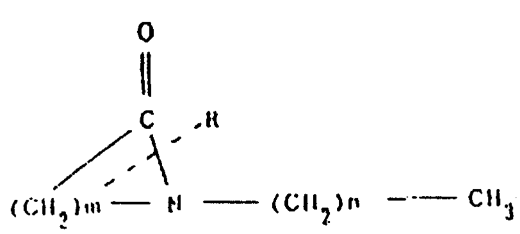

dans laquelle R est H ou un groupe alkyle inférieur, n est de 5 à 7 et n est de 0 à 17, des acides gras saturés et insaturés et leurs esters d'alkyle, des alcools, des monoglycérides, des diéthanolamides, des complexes de triéthanolamino, et des dérivés de N, N-diméthylamide, et des esters d'acétate desdits

dérivés d'alcools; le myristate de propyle, l'alcool myristylique; l'oléate de propyle; l'acide oléique; l'alcool oléylique; l'acétate d'oléyle; la monooléine; le NN-diméthyl amide d'acide myristique; l'acide stearique; l'alcool stearilique ie stéarate de propyle; la monostéarine; la mono-myristéine; l'acide salicylique et ses dérivés; la glycylglycine; le N,N-diéthyl-m-toluamide; le crotamiton; et les combinaisons de ceux-ci.

**9.** Unité de dosage à matrice polymère contenant un produit phamaceutique transdermique selon l'une quelconque des revendications 1 à 8 dans laquelle la couche adhésive comporte environ 1 à environ 30% d'un ou plusieurs agents d'amélioration de l'absorption de la peau distribués en son sein par rapport au poids de l'adhésif de polysiloxane.

**10.** Unité de dosage à matrice polymère contenant un produit pharmaceutique transdermique selon l'une quelconque des revendications 1 à 9 dans laquelle le Facteur d'Amélioration tel que défini précédemment est d'au moins environ 2.

**11.** Unité de dosage à matrice polymère contenant un produit pharmaceutique transdermique selon l'une quelconque des revendications 1 à 10 qui comporte une couche protectrice pouvant être éliminée et adhérant à la couche adhésive.

**12.** Unité de dosage à matrice polymère contenant un produit pharmaceutique transdermique selon l'une quelconque des revendications 1 à 11 dans laquelle le produit pharmaceutique présente une activité anti-anginale, anti-arrhytmique, anti-hypertensive, tranquilisante, diurétique, sur le système nerveux central, antiinflammatoire, analgésique ou anti-arthritique.

**13.** Unité de dosage à matrice polymère contenant un produit pharmaceutique transdermique selon l'une quelconque des revendications 1 à 12 dans laquelle le produit pharmaceutique est un stéroïde.

**14.** Unité de dosage à matrice polymère contenant un produit pharmaceutique transdermique selon la revendication 13, dans laquelle le produit pharmaceutique comprend la testostérone, l'estradiol, la progestérone, l'hydrocortisone ou l'indométhacine.

**15.** Unité de dosage à matrice polymère contenant un produit pharmaceutique transdermique selon l'une quelconque des revendications 1 à 12 dans laquelle le produit pharmaceutique est la nitroglycérine.

**16.** Procédé de production d'une unité de dosage à matrice polymère contenant un produit pharmaceutique transdermique ayant un Facteur d'Amélioration tel que défini dans la revendication 1 d'au moins 1,2, ce procédé comprenant :

(a) l'adhérence d'une face de la couche de motrice polymère en forme de disque comportant une quantité du produit pharmaceutique en microdispersion dans son sein constituant une quantité de dosage du produit pharmaceutique à administrer par voie transdermique, à une couche de renforcement qui est substantiellement imperméable audit produit pharmaceutique, et

(b) l'adhérence de l'autre face de ladite couche de matrice polymère en forme de disque à une couche adhésive comportant une quantité efficace d'un ou plusieurs agents d'amélioration de l'absorption de la peau distribuée en son sein et procurant une amélioration d'absorption de la peau substantielle pour ledit produit pharmaceutique.

**Patentansprüche**

**1.** Transdermale, ein Arzneimittel enthaltende Polymermatrix-Dosierungseinheit umfassend:

a) eine Verstärkungsschicht, die im wesentlichen gegenüber dem transdermal abzugebenden Arzneimittel undurchlässig ist;

b) eine Polymermatrix-Lamellenschicht, die an der Verstärkungsschicht haftet und die mikrodispergiert hierin eine Menge des Arzneimittels enthält, die eine Dosierungsmenge des transdermal abzugebenden Arzneimittels liefert; und

c) eine Haftschicht, die an der das Arzneimittel enthaltenden Polymermatrix-Lamellenschicht haftet und hierin verteilt eine wirksame Menge eines oder mehrerer Hautabsorbtionsverstärker enthält, die eine erhebliche Hautabsorbtionsverstärkung für das Arzneimittel ergeben;

wobei die Dosierungseinheit einen Verstärkungsfaktor, definiert als das Verhältnis der normierten

Permeationsrate (in $mcg/cm^2/h$) einer Dosierungseinheit mit Hautabsorbtionsverstärker zur normierten Permeationsrate einer Dosierungseinheit ohne Verstärker, von zumindest 1,2 besitzt.

**2.** Transdermale, ein Arzneimittel enthaltende Polymermatrix-Dosierungseinheit gemäß Anspruch 1, worin die Polymermatrix-Lamellenschicht ein vernetztes Polysiloxan-Polymeres der folgenden Formel:

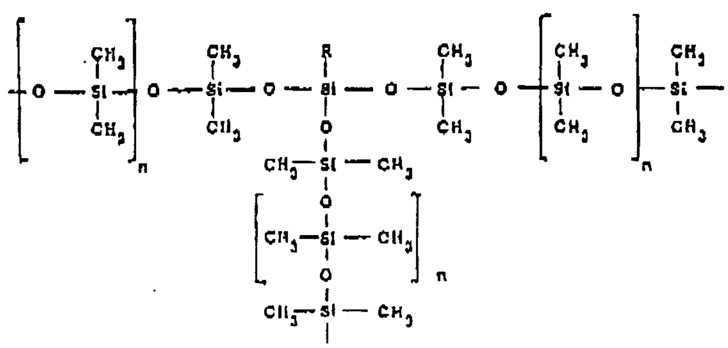

ist, worin R für Alkyl oder Alkoxy mit 1 bis 7 Kohlenstoffatomen, Vinyl, Phenyl oder eine Kombination hiervon steht und worin n etwa 100 bis etwa 5000 beträgt.

**3.** Transdermale, ein Arzneimittel enthaltende Polymermatrix-Dosierungseinheit gemäß Anspruch 2, worin R in der Polysiloxanformel für Propoxy steht.

**4.** Transdermale, ein Arzneimittel enthaltende Polymermatrix-Dosierungseinheit gemäß einem der Ansprüche 1 bis 3, worin die Polymermatrix-Lamellenschicht eine Dicke von 0,05 bis 5 mm aufweist.

**5.** Transdermale, ein Arzneimittel enthaltende Polymermatrix-Dosierungseinheit gemäß einem der Ansprüche 1 bis 4, worin die Polymermatrix-Lamelle Mikrodispersionsabteilungen mit einer Querschnittsabmessung von etwa 10 bis etwa 200 Mikron ergibt.

**6.** Transdermale, ein Arzneimittel enthaltende Polymermatrix-Dosierungseinheit gemäß einem der Ansprüche 1 bis 5, worin die Haftschicht aus einem druckempfindlichen Haftmittel besteht.

**7.** Transdermale, ein Arzneimittel enthaltende Polymermatrix-Dosierungseinheit gemäß einem der Ansprüche 1 bis 6, worin die Haftschicht aus einem Silikonpolymeren-Haftmittel besteht.

**8.** Transdermale, ein Arzneimittel enthaltende Polymermatrix-Dosierungseinheit gemäß einem der Ansprüche 1 bis 7, worin der Hautabsorbtionsverstärker ausgewählt ist unter Decylmethylsulfoxid, 1-Dodecylazacycloheptan-2-on, einer Verbindung der Formel:

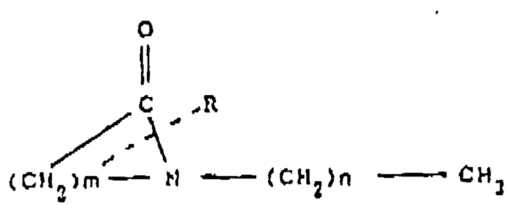

worin R für H oder eine Niedrigalkylgruppe steht, n für 5 bis 7 steht und n 0 bis 17 ist, gesättigten und ungesättigten Fettsäuren und deren Alkylestern, Alkoholen, Monoglyceriden, Diethanolamiden, Triethanolamin-Komplexen und N,N-Dimethylamid-Derivaten und Acetatestern dieser Alkoholderivate, Propylmyristat, Myristylalkohol, Propyloleat, Ölsäure, Oleylalkohol, Oleylacetat, Monoolein, Myristyl-N,N-dimethylamid, Stearinsäure, Sterarylalkohol, Propylstearat, Monostearin, Monomyristein, Salicylsäure und Derivaten, Glycylglycin, N,N-Diethyl-m-toluamid, Crotamiton und Kombinationen hiervon.

**9.** Transdermale, ein Arzneimittel enthaltende Polymermatrix-Dosierungseinheit gemäß einem der Ansprüche 1 bis 8, worin die Haftschicht hierin verteilt etwa 1 bis etwa 30 % eines oder mehrerer

Hautabsorbtionsverstärker, bezogen auf das Gewicht des Polysiloxanhaftmittels, enthält.

**10.** Transdermale, ein Arzneimittel enthaltende Polymermatrix-Dosierungseinheit gemäß einem der Ansprüche 1 bis 9, worin der Verstärkungsfaktor wie vorliegend definiert, zumindest etwa 2,0 beträgt.

**11.** Transdermale, ein Arzneimittel enthaltende Polymermatrix-Dosierungseinheit gemäß einem der Ansprüche 1 bis 10, die eine freisetzbare, an der Haftschicht anhaftende Schutzschicht aufweist.

**12.** Transdermale, ein Arzneimittel enthaltende Polymermatrix-Dosierungseinheit gemäß einem der Ansprüche 1 bis 11, worin das Arzneimittel anti-anginale, anti-arrythmische, anti-hypertensive Wirkung, Tranquilizerwirkung, diuretische Wirkung, Wirkung auf das Zentralnervensystem, anti-inflammatorische, analgetische oder anti-arthritische Wirkung besitzt.

**13.** Transdermale, ein Arzneimittel enthaltende Polymermatrix-Dosierungseinheit gemäß einem der Ansprüche 1 bis 12, worin das Arzneimittel ein Steroid ist.

**14.** Transdermale, ein Arzneimittel enthaltende Polymermatrix-Dosierungseinheit gemäß Anspruch 13, worin das Arzneimittel Testosteron, Östradiol, Progesteron, Hydrocortison oder Indometacin umfaßt.

**15.** Transdermale, ein Arzneimittel enthaltende Polymermatrix-Dosierungseinheit gemäß einem der Ansprüche 1 bis 12, worin das Arzneimittel Nitroglycerin ist.

**16.** Verfahren zur Herstellung einer transdermalen ein Arzneimittel enthaltenden Polymermatrix-Dosierungseinheit mit einem Verstärkungsfaktor wie in Anspruch 1 definiert von zumindest 1,2, umfassend:
a) das Anbringen einer Seite einer Polymermatrix-Lamellenschicht, die hierin mikrodispergiert eine Menge des Arzneimittels enthält, die eine Dosierungsmenge des transdermal freizugebenden Arzneimittels ergibt, an eine Verstärkungsschicht, die gegenüber dem Arzneimittel im Wesentlichen undurchlässig ist und
b) das Anbringen der anderen Seite dieser Polymermatrix-Lamellenschicht an eine Haftschicht, die hierin verteilt eine wirksame Menge eines oder mehrerer Hautabsorbtionsverstärker enthält, die eine erhebliche Hautabsorbtionsverstärkung für dieses Arzneimittel ergeben.